# EUROPÄISCHE PATENTSCHRIFT

(11) **EP 1 478 699 B1**
(45) Veröffentlichungstag und Bekanntmachung des Hinweises auf die Patenterteilung: **04.06.2008**
(21) Anmeldenummer: 03743345.5
(22) Anmeldetag: 27.02.2003
(51) Int. Cl.: C09C 1/00

(54) **PLÄTTCHENFÖRMIGE EFFEKTPIGMENTE MIT EINER BESCHICHTUNG AUS MELAMIN-FORMALDEHYD-HARZEN**
PLATELIKE EFFECT PIGMENTS WITH A MELAMINE FORMALDEHYDE RESIN COATING
PIGMENTS A EFFET SOUS FORME DE PLAQUETTES POURVUS D'UN REVETEMENT EN RESINE MELAMINE-FORMALDEHYDE

(30) Priorität: 02.03.2002 DE 10209359; 08.10.2002 EP 02022552
(43) Veröffentlichungstag der Anmeldung: 24.11.2004
(73) Patentinhaber: Merck Patent GmbH, 64293 Darmstadt (DE)
(72) Erfinder: KÜBELBECK, Armin, 64625 Bensheim (DE); ANSELMANN, Ralf, 67305 Ramsen (DE); EICHHORN, Jens, 64354 Reinheim (DE)
(74) Vertreter: Leifert & Steffan
(86) Internationale Anmeldenummer: PCT/EP2003/002020
(87) Internationale Veröffentlichungsnummer: WO 2003/074614

(56) Entgegenhaltungen:
- EP-A- 0 280 749
- EP-A- 0 601 378
- DD-A- 224 602
- DE-A- 4 317 019
- GB-A- 858 072
- GB-A- 974 874

## Beschreibung

Die Erfindung betrifft mit gehärteten Melamin-Formaldehyd-Harzeri beschichtete plättchenförmige Substrate, ein Verfahren zu deren Herstellung, sowie deren Verwendung als Effektpigmente.

Die Verwendung plättchenförmiger Trägermaterialen, wie zum Beispiel Glimmerplättchen, Silicaflakes, Glasplättchen oder Aluminiumflakes zur Herstellung von Effektpigmenten, wie zum Beispiel Schuppenpigmenten, ist allgemein bekannt. Die Trägermaterialien dienen hierbei als Template für die Abscheidung von zumeist anorganischen Oxidschichten, wie zum Beispiel Titandioxid, Zirkoniumdioxid oder Eisenoxiden, welche in bestimmten Fällen mit dem Substrat durch Interferenz optisch in Wechselwirkung treten können. Derartige Pigmente sind zum Beispiel aus DE 1467468, DE 1959998 und DE 2009566 bekannt. Die Herstellung von Silicaflakes sowie Silicaflakes selbst sind in WO 93/08237 und WO 92/02351 beschrieben. Aus EP 0810270 sind Pigmente auf Aluminiumbasis bekannt, die mit einer sauren Adsorptionsschicht und einer darauf befindlichen Farbpigmentschicht versehen sind.

Eine Beschichtung oder Verkapselung von Oberflächen mit Melaminharzen ist ebenfalls aus der Literatur bekannt, so ist zum Beispiel in DE 19710619 das Beschichten von festen, harten Partikeln mit Melaminharz für dekorative, abriebfeste Beschichtungen beschrieben.

EP 601378 offenbart einen Lack auf Wasserbasis, in welchem sich die Beschichtung von Glimmerflocken mit ungehärtetem Melaminharz als günstig für die Verarbeitbarkeit des Lacks und die Stabilität der Dispersion erweist.

Eine verbesserte Benetzbarkeit von Tonerpartikeln, welche mit verschiedenen Harzen, unter anderem mit Melaminharz beschichtet sind, wird in JP 130787 beschrieben.

In DD 224 602 und EP 445 342 werden säurevernetzte und ausgefällte Melaminharzpartikel offenbart, in welche wasserlösliche Amino- oder Sulfonsäuregruppen enthaltende Farbstoffe eingebaut werden können.

Eine der Aufgaben der vorliegenden Erfindung bestand darin Effektpigmente bereitzustellen, die einfärbbar sind, eine hohe Brillanz aufweisen und Im Falle kosmetischer Anwendungen einen hohen Hautkomfort besitzen.

Diese Aufgabe wurde durch die Bereitstellung eines beschichteten plättchenförmigen Trägermaterials gelöst, wobei das Trägermaterial aus einem anorganischen Substrat besteht und das Trägermaterial mit mindestens einer Beschichtung versehen ist, wobei jede Schicht mindestens ein gehärtetes Melamin-Formaldehyd-Harz enthält oder aus einem solchen besteht, und wobei das gehärtete Melamin-Formaldehyd-Harz einen oder mehrere anorganische oder organische Farbstoffe und/oder einen oder mehrere anorganische oder organische UV-Absorber enthält, wobei die Farbstoffe im Medium, In welchem das Pigment beschichtet wird, löslich sind.

Prinzipiell sind als Substrat alle anorganischen plättchenförmigen Trägermaterialien geeignet. Unter einem plättchenförmigen Substrat, ist ein Substrat mit einer im Wesentlichen flachen, insbesondere blättchen- oder schuppenartigen Form zu verstehen. Hierbei kommen zum Beispiel Glimmer, wie Muskovit, Phlogoplt, Fluorphlogopit oder andere silikatische Plättchen, Silica- oder Glasflakes, aber auch Metallflakes oder -follen, wie zum Beispiel solche aus Silber, Kupfer, Nickel, Gold, Aluminium oder Legierungen dieser Metalle In frage. Insbesondere eignen sich als plättchenförmige anorganische Substrate auch Periglanzpigmente, die aus beispielsweise Glimmer, Silicaflakes oder Glas und einer oder mehreren darauf abgeschiedenen Metalloxidschichten bestehen. Die Metalloxidschicht kann beispielsweise aus Titandioxid. Titandioxid im Gemisch mit Eisen(III)oxid, Eisen(III)oxid, Chromoxid, Zirkondioxid, Zinndioxid oder Zinkoxid bestehen. Derartige Pigmente sind im Handel unter der Bezeichnung Iriodin® (Hersteller E. Merck, Darmstadt) erhältlich.

Die metallischen wie auch die nicht-metallischen Trägermaterialien können eine metallische Beschichtung mit den oben genannten Metallen oder deren Legierungen besitzen.

Melaminmoleküle auch durch andere vernetzbare Moleküle, wie zum Beispiel Phenole, Guanamine oder Harnstoff ersetzt sein. Die Melamin-Formaldehyd-Harze können unveretherte oder veretherte Melamin-Formaldehydaddukte, zum Beispiel Alkoxymethylol-Melamine mit C₁-C₆-Alkoxygruppen, wie Methoxy- oder n-Butoxy-Gruppen, und Prekondensate sein. Beispielhaft kann als unverethertes Harz Madurit MW 909 oder als verethertes Harz Madurit SMW 818 (beides Produkte der Firma Solutia, Wiesbaden , Deutschland) genannt werden. Ein Teil des Melamin-Formaldehyd-Harzes kann auch durch andere vernetzende organische Polymere ersetzt werden. Hierbei eignen sich insbesondere solche, die ebenfalls eine hohe Brechzahl besitzen, ganz besonders solche, die eine Brechzahl besitzen, die größer als die des Substrats ist.

In die Melamin-Formaldehyd-Harze können beliebige organische und anorganische Farbstoffe sowie auch gegebenenfalls farblose UV-Absorber eingebaut werden. Ausschlaggebend für den Einbau in die Polymermatrix ist hierbei nur deren Löslichkeit im Medium, in dem die Beschichtungsreaktion durchgeführt wird. Selbst wasserlösliche Farbstoffe, wie zum Beispiel Eosin, Fluorescein oder Victoria Pure Blue BO lassen sich in die Polymermatrix einbetten ohne später auszubluten. Bei lipophilen Farbstoffen kann die Beschichtungsreaktion ebenfalls in wässrigem Medium durchgeführt werden, wenn die dem Durchschnittsfachmann auf dem Gebiet geläufigen Lösungsvermittler zugegeben werden. Beispielhaft für einen Lösungsvermittler kann hierbei 1-Methyl-2-pyrrolidon genannt werden.

Um Farbnuancen zu erhalten, kann man sich den üblichen Prinzipien additiver Farbmischungen bedienen. Hierbei können die Farbtöne eingestellt werden, indem die Farbstoffe vorab gemischt und gemeinsam in eine Polymerschicht eingebracht werden oder indem mehrere Farbstoff-Polymer-Schichten auf das anorganische Substrat nacheinander aufgebracht werden, sodass sich Schichten unterschiedlicher Farbe überlagern.

Auch acidochrome Farbstoffe, das heißt Farbstoffe, deren Farbe vom pH-Wert abhängt, können im Wesentlichen unter Erhalt von Farbton und Farbumschlagpunkt in das Melamin-Formaldehyd-Harz eingebaut werden. Exemplarisch können hier werden.

Neben dem bereits oben genannten Fluorescein lassen sich auch andere Fluoreszenz-Farbstoffe, optische Aufheller oder andere UV-Licht absorbierende Farbstoffe in die Polymermatrix einbauen.

Insbesondere empfiehlt sich der Einbau mehrerer Farbstoffe, von denen mindestens ein Farbstoff ein Fluoreszenzfarbstoff ist. Besonders vorteilhaft ist der Einbau von mindestens zwei Fluoreszenzfarbstoffen, wobei der zweite Fluoreszenzfarbstoff in erheblich geringeren Mengen zugesetzt wird. Hierdurch können Pigmente erhalten werden, deren resultierende Fluoreszenzfarbe deutlich von der Fluoreszenzfarbe der Ausgangsstoffe abweicht. Auf diese Weise ist es möglich, eine Vielzahl unterschiedlich fluoreszierender Pigmente auf einfache Weise zu synthetisieren. Durch Variation der Art des oder der Fluoreszenzfarbstoffe und Variation des zweiten, in erheblich geringeren Mengen zugesetzten Farbstoffs, sowohl hinsichtlich des Typs als auch der Konzentration, kann eine große Bandbreite an Fluoreszenzfarben erzeugt werden. Häufig wirken diese Pigmente im sichtbaren Licht durch den geringen Anteil an farbbestimmenden Farbstoffen sehr unscheinbar und vergleichsweise blaß. Neben den Grundfarben Rot, Grün und Blau können weit über hundert verschiedene mit dem Auge deutlich unterscheidbare Fluoreszenzfarben realisiert werden. Die auf diese Weise erhältliche Vielfalt an Fluoreszenzpigmenten kann beispielsweise zur Kodierung von Produkten wie z. B. Saatgut verwendet werden. Die Kodierungsmöglichkeiten gehen jedoch weit über die mit dem Auge erfaßbaren Unterscheidungsmöglichkeiten hinaus. Insbesondere der Einsatz von Fluoreszenzdetektoren eröffnet die Möglichkeit einer stark nuancierten Kodierung bzw. Markierung von Produkten. So läßt sich beispielsweise durch die Variation der Konzentration eines bestimmten Farbpigments einerseits sowie der Anzahl der insgesamt eingesetzten Farbpigmente andererseits eine Vielzahl von Farbnuancen erreichen, die letztlich nur durch praktische Erwägungen begrenzt ist. So lassen sich beispielsweise beim Einsatz von fünf Farbpigmenten, die in jeweils acht diskreten Konzentrationsstufen vorliegen, bereits über 4000 Kodierungsmöglichkeiten erhalten. Bei zehn Fluoreszenzfarben und vierzehn Konzentrationen ergeben sich gar über 20 Milliarden Kodierungsmöglichkeiten. Dies Pigmentpartikeln eingesetzt wird, was z. B. durch eine große, zur Messung und Auswertung zur Verfügung stehende Fläche oder durch sehr kleine Pigmentpartikel und hoch empfindliche Detektoren erreicht werden kann. Markierungen unter Verwendung von mehreren Farbpigmenten in unterschiedlichen Konzentrationsstufen können für verschiedene Anwendungen genutzt werden. Beispielsweise kann ein Hersteller von Gebrauchsartikeln auf seinen Produkten verschiedene Informationen wie z. B. das Herstelljahr und die Chargennummer durch Bedrucken mit einem Lack, welcher die beschriebenen Fluoreszenzpigmente enthält, unterbringen. Derartige Informationen können unter anderem auch dem Produktschutz dienen, indem eigene Produkte schnell identifiziert werden können und sich somit von Produkten nicht autorisierter Hersteller oder Vertreiber (Produktpiraterie) unterscheiden lassen.

Durch Abscheiden einer einen oder mehrere Fluoreszenzfarbstoffe enthaltenden Polymerschicht auf einer bereits vorher aufgebrachten Farbstoff enthaltenden Polymerschicht lassen sich Brillanz und Leuchtkraft der Effektpigmente deutlich steigern. Darüber hinaus kann ein Ausbleichen der darunter liegenden Schichten durch die Absorption des UV-Lichts gehemmt werden. Ein derartiger UV-Schutz kann auch durch den Einbau von UV-Absorbern in die Farbstoff enthaltende Polymerschicht selbst erreicht werden.

Als geeignete UV-Absorber kommen prinzipiell alle UV-Filter in Frage. Besonders bevorzugt sind solche UV-Filter, deren physiologische Unbedenklichkeit nachgewiesen ist. Sowohl für UV-A- wie auch für UV-B-Filter gibt es viele aus der Fachliteratur bekannte und bewährte Substanzen. Hierbei sind beispielsweise Benzylidenkampferderivate, wie 3-(4'-Methylbenzyliden)-di-kampfer, 3-Benzylidenkampfer, Polymere des N-{(2 und 4)-[(2-oxoborn-3-yliden)methyl]benzyl}-acrylamid, N,N,N-Trimethyl-4-(2-oxoborn-3-ylidenmethyl)anilinium methylsulfat oder α-(2-Oxoborn-3-yliden)toluol-4-sulfonsäure, Benzoyl- oder Dibenzoylmethane wie zum Beispiel 1-(4-tert-Butylphenyl)-3-(4-methoxyphenyl)propan-1,3-dion oder 4-Isopropyldibenzoylmethan,
Benzophenone wie zum Beispiel 2-Hydroxy-4-methoxybenzophenon oder 2-Hydroxy-4-methoxybenzophenon-5-sulfonsäure und deren Natriumsalz, Methoxyzimtsäureisopentylester und dessen Isomerengemisch, Salicylatderivate wie zum Beispiel
2-Ethylhexylsalicylat, 4-Isopropylbenzylsalicylat oder
3,3,5-Trimethylcyclohexylsalicylat,
4-Aminobenzoesäure und deren Derivate wie 4-(Dimethylamino)benzoesäure-2-ethylhexylester oder
ethoxylierter 4-Aminobenzoesäureethylester, sowie weitere Substanzen wie zum Beispiel 2-Cyano-3,3-diphenylacrylsäure-2-ethylhexylester, 2-Phenylbenzimidazol-5-sulfonsäure sowie deren Kalium-, Natrium- und Triethanolaminsalze, 3,3'-(1,4-Phenylendimethylen)-bis-(7,7-dimethyl-2-oxobicyclo-[2.2.1]hept-1-ylmethansulfonsäure sowie deren Salze und
2,4,6-Trianilino-(p-carbo-2'-ethylhexyl-1'-oxi)-1,3,5-triazin, zu nennen.

Weitere geeignete organische UV-Filter sind zum Beispiel
2-(2H-Benzotriazol-2-yl)-4-methyl-6-(2-methyl-3-(1,3,3,3-tetramethyl-1-(trimethylsilyloxy)disiloxanyl)propyl)phenol,
4,4'-[(6-[4-((1,1-Dimethylethyl)aminocarbonyl)phenylamino]-1,3,5-triazin-2,4-diyl)diimino]bis(benzoesäure-2-ethylhexylester), α-(Trimethylsilyl)-ω-[trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1), 2,2'-Methylen-bis-(6-(2H-benzotriazol-2-yl)-4-(1,1,3,3-tetramethylbutyl)phenol), 2,2'-(1,4-Phenylen)bis-(1H-benzimidazol-4,6-disulfonsäure, Mononatriumsalz) und 2,4-bis-{[4-(2-Ethyl-hexyloxy)-2-hydroxyl]-phenyl)-6-(4-methoxyphenyl)-1,3,5-triazin.

Bevorzugte Verbindungen mit UV-absorbierenden Eigenschaften sind 3-(4'-Methylbenzyliden)-dl-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethyl-cyclohexylsalicylat, 4-(Dimethylamino)benzoesäure-2-ethylhexylester, 2-Cyano-3,3-diphenylacryasäure-2-ethylhexylester, 2-Phenylbenzimidazol-5-sulfonsäure sowie deren Kalium-, Natrium- und Triethanolaminsalze.

Durch Kombination von mehreren UV-Filtern kann die Schutzwirkung gegen schädliche Einwirkungen der UV-Strahlung optimiert werden.

Die beschichteten plättchenförmigen Substrate lassen sich durch Abscheiden vernetzender Melamin-Formaldehyd-Harze auf den suspendierten plättchenförmigen Substraten und anschließendem Aushärten, das heißt Vernetzen der Melamin-Formaldehyd-Harze herstellen.

Das erfindungsgemäße Verfahren zur Herstellung des erfindungsgemäßen einfach oder mehrfach beschichteten plättchenförmigen Trägermaterials, umfasst bei einer Einfachbeschichtung
einen ersten Schritt bei welchem ein anorganisches plättchenförmiges Substrat in basischem wässrigem Milieu, enthaltend Melamin und Formaldehyd und/oder Methylolmelamin, welches gegebenenfalls alkoxyliert sein kann, suspendiert wird und
einen zweiten Schritt in welchem durch Senkung des pH-Werts in den sauren Bereich eine Vernetzung der organischen Bestandteile herbeigeführt wird
und bei einer Mehrfachbeschichtung
der erste und zweite Schritt mit dem Produkt des vorangegangenen Beschichtungsvorgangs wiederholt wird.

Es hat sich herausgestellt, dass es besonders vorteilhaft ist, die pH-Wert-Senkung Im zweiten Verfahrensschritt durch Zugabe von Wasserstoffperoxid zu bewirken, indem überschüssiger oder nicht umgesetzter Formaldehyd aus dem ersten Verfahrensschritt zu Ameisensäure oxidiert wird. Da Formaldehyd in kosmetischen Anwendungen problematisch ist, kann somit ein Pigment zur Verfügung gestellt werden, welches frei von freien Formaldehydmolekülen und somit kosmetisch unbedenklich ist. Dies funktioniert auch mit Methylolmelaminen, da diese meist noch ausreichende Mengen an freiem Formaldehyd enthalten.

Im erfindungsgemäßen Verfahren kann ein Teil des Melamins durch andere vernetzende Moleküle aus der Gruppe bestehend aus "Guanaminen, Phenolen und Guanamin-, Phenol- oder Harnstoffanaloga ersetzt werden.

Vor Einsetzen oder während der Vernetzungsreaktion können anorganische oder organische Farbstoffe und/oder anorganische oder organische UV-Absorber zugesetzt werden.

Sollten sich die Farbstoffe oder UV-Absorber im wässrigen Milieu nicht vollständig lösen, so kann eine vollständige Lösung durch Lösungsvermittler herbeigeführt werden. Dies gilt insbesondere bei der Verwendung von lipophilen Substanzen.

Die Schichtdicke der.Beschichtung lässt sich hierbei durch die Melamin-Formaldehyd-Harz-Konzentration steuern. So werden bei hohen Konzentrationen größere Schichtdicken erhalten als bei niedrigen Konzentrationen. Auch der pH-Wert ist ein geeignetes Mittel zur Steuerung der Schichtdicke. Niedrige pH-Werte führen zu dünneren Beschichtungen. Die DE 1595386 beschreibt darüber hinaus die Steuerung von Schichtdicken durch den Zusatz von Schutzkolloiden.

Bevorzugte Gesamtschichtdicken von einfach oder mehrfach beschichteten Substraten betragen vorzugsweise 0,2 µm bis 4 µm*.*

Durch Verwendung von überschüssigem Melamin-Formaldehyd-Harz können auf der äußersten Beschichtung zusätzliche, im Wesentlichen runde Melamin-Formaldehydharzpartikel abgeschieden werden, die neben organischen Farbstoffen auch UV-Absorber enthalten können oder gänzlich frei von Farbstoffen oder UV-Absorbern sind.

Je nach Reaktionsführung kann das Verhältnis von Kugeln zu Plättchen, der Kugeldurchmesser, sowie die Verteilung der Kugeldurchmesser (Dispersität) gesteuert werden. Insbesondere für kosmetische Zwecke ist ein gewisser Anteil an Kugeln für ein verbessertes Hautgefühl vorteilhaft.

Erscheinungsbild des Pigments. Bei ausreichendem Anteil an Farbstoff weisen die zusätzlichen Kugeln eine zu den Plättchen passende Färbung auf.

Die unter dem Gesichtpunkt der Wirtschaftlichkeit relevante Raum-Zeit-Ausbeute kann durch Zusatz von Polymeren mit stark sauren Gruppen deutlich gesteigert werden, wie dies zum Beispiel in EP 0415273 beschrieben ist.

Die beschichteten plättchenförmigen Substrate eigenen sich hervorragend als Effektpigmente und können in allen üblicherweise Effektpigmente enthaltenden Systemen eingesetzt werden.

Somit sind diese einerseits in (Druck-)Farben, Lacken, Kunststoffen und Pulverlacken einsetzbar, andererseits eignen sich die Effektpigmente auch zur Saatguteinfärbung als Saatbeize, im Lebensmittelbereich zur Veredlung von Lebensmittel bzw. dem Lebensmitteldesign oder im kosmetischen Bereich zum Beispiel in Make-Ups, Lippenstiften oder Sonnenschutzformulierungen.

Für bestimmte Anwendungen, zum Beispiel in Lacken, Farben und dergleichen kann es vorteilhaft sein in das Kondensationsprodukt der äußersten Schicht des Polymers auch andere funktionelle Gruppen als die genannten stark sauren Gruppen einzubauen, um diese zum Beispiel hinsichtlich ihrer Bindemittelkompatibilität und des Dispersionsverhaltens zu verbessern. Auch ein nachträgliches Versehen der äußersten Schicht des vernetzten organischen Polymers mit funktionellen Gruppen durch nachträgliches Zurreaktionbringen der Melamin-Formaldehyd-Harze ist möglich. Die DD 224 602 beschreibt verschiedene Möglichkeiten der Funktionalisierung von Harzen.

Funktionelle Gruppen im Sinne dieser Erfindung können beliebige hydrophile oder hydrophobe, saure oder basische Gruppen sein, so fallen hierunter z. B. auch rein hydrophobe weitgehend inerte Gruppen wie z. B. Alcylgruppen.

Nach einem der in DD 224 602 beschriebenen Verfahren erfolgt der Einbau funktioneller Gruppen in die Oberfläche der Polymerteilchen, in dem die aminofunktionellen Verbindungen erfolgt, wobei die aminofunktionellen Verbindungen neben der Aminogruppe weitere funktionelle Gruppen tragen. Die aminofunktionellen Verbindungen werden in Mengen von vorzugsweise 2 bis 20 Molprozent bezogen auf die eingesetzte Menge des Methylolmelamins zugegeben und über die Aminofunktion in das Melamin-Formaldehyd-Netzwerk eingebaut. So lassen sich z. B. beim Einsatz von Aminosäuren-Carboxylgruppen oder im Falle der Sulfobetaine oder Aminophosphonsäuren Sulfo- bzw. Phosphonsäuregruppierungen in die Oberfläche der Teilchen einbauen. Derartige -COOH, -SO₃H bzw. -PO₂H-Gruppen können wiederum mit anderen Verbindungen umgesetzt werden. Beispielsweise können die Säuregruppierungen durch Umsetzung mit Tionylchlorid in entsprechende Säurechloride überführt werden, die beispielsweise wiederum mit Alkoholen oder Aminen umsetzbar sind, wobei die korrespondierenden Ester bzw. Amide entstehen. Dieses Verfahren der Oberflächenmodifizierung ist durch seine Einfachheit gekennzeichnet, da in einem nur leicht modifizierten Kondensationsprozess unmittelbar eine Funktionalisierung der Melamin-Formaldehyd-Harz-Oberfläche erfolgt. Nachteilig kann sich jedoch auswirken, dass durch den Kondensationsprozess die entsprechenden Funktionalitäten auch im Polymervolumen eingebaut werden und damit die Haftung zu den darunter liegenden Schichten oder im Falle eines einschichtigen Aufbaus die Haftung zum Substrat vermindert werden kann. Andererseits kann jedoch auch bei einer entsprechenden Auswahl für bestimmte Systeme die Haftung zu den darunter liegenden Schichten bzw. zum Substrat erhöht werden, wenn durch das Oberflächenmodifizierungsagens Gruppen eingebracht werden, die sowohl die Verträglichkeit zum Umgebungsmedium verbessern als auch eine Haftung an die darunter liegenden Schichten bzw. das Substrat vermitteln. Bei diesem Verfahren werden jedoch durch den Einbau in das Melamin-Formaldehyd-Netzwerk relativ große Mengen des oberflächenfunktionalisierenden Agens benötigt. Auch lassen sich komplexere chemische Funktionalitäten durch einen einfachen Einbau während der Polykondensation nur schwer erhalten.

Ein anderes Verfahren zur Oberflächenfunktionalisierung geht daher von einer fertig polykondensierten Melamin-Formaldehyd-Oberfläche aus, welche freie, nicht vernetzte Methylolamin- (NH-CH₂OH-) oder Amino-Gruppen aufweist. Diese umgesetzt werden. So kann z. B. beim Einsatz langkettiger Carbonsäurechloride eine Hydrophobisierung des Pigments erreicht werden. Mit perfluorierten Säurechloriden wie z. B. Per-Fluoroctansäure können sowohl hydrophobe als auch lipophobe Oberflächen erhalten werden. Durch den Einsatz komplexer Säurechloride; welche z. B. stark UV-Licht absorbierende Gruppen enthalten können, kann die Melamin-Formaldehyd-Oberfläche auch weitergehende Funktionalitäten, z. B. einen UV-Schutz enthalten. Ein Beispiel für die nachträgliche Hydrophobisierung einer Pigmentoberfläche ist in Beispiel 9 dargestellt.

Die vorliegende Erfindung soll anhand der folgenden Beispiele verdeutlicht werden.

### Beispiel 1

0,63 g 2,4,6-Triamino-1,3,5-triazin (Melamin) werden in 50 ml Wasser einer Temperatur von 70 °C gelöst. Anschließend werden 0,05 ml Tetramethylammoniumhydroxid (25 gew.-%ig) und 2,43 g Formaldehyd-Lösung (37 gew.-%ig) unter Rühren zugegeben. Die Formylierungsreaktion findet bei einem pH-Wert von 9,5 statt. Die klare Lösung wird 10 min gerührt, worauf 1,51 g Glimmer (durchschnittliche Teilchengröße 15 µm) zugegeben werden. Nach einer Minute werden der Suspension 0,3 ml Wasserstoffperoxid (30 gew.-%ig) zugetropft, um freies Formaldehyd zu Ameisensäure zu oxidieren. Der pH-Wert fällt gleichmäßig bis auf ca. 3,5 ab, worauf nach ca. 5 min die Kondensationsreaktion des Melaminharzes einsetzt. Es wird weitere 15 min zur Vervollständigung der Reaktion gerührt. Die Reaktionstemperatur wird während der gesamten Zeitdauer bei 70 °C gehalten. Danach wird der beschichtete Glimmer abfiltriert, mit Wasser gewaschen und im Trockenschrank bei 105 °C für 1 h getrocknet.

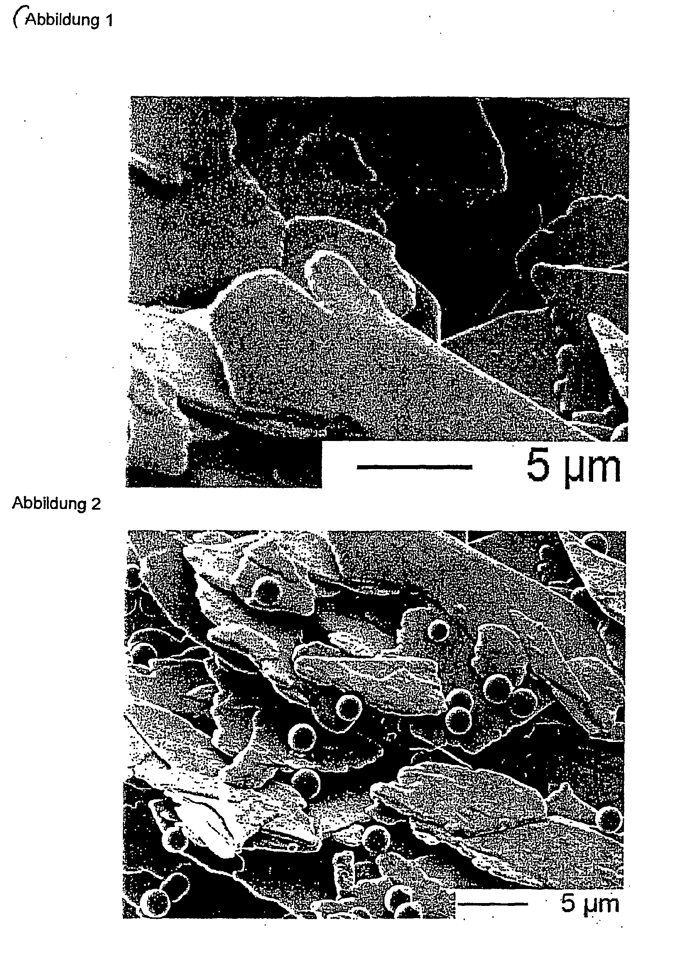

Das so erhaltene Produkt zeigt beim Verreibetest zwischen den fingern ein weicheres, angenehmeres Hautgefühl, als der unbelegte Glimmer, welcher auf der Haut einen stumpfen Eindruck hinterlässt. Die Kanten sind, wie Abbildung 1 zeigt, abgerundet.

Optisch hat der Glimmer eine etwas höhere Brillanz als der unbeschichtete Glimmer.

### Beispiel 2

Beispiel 2 wird in Analogie zu Beispiel 1 durchgeführt, außer dass zusätzlich, gleichzeitig mit der Zugabe von Melamin 0,05 g Allura ® Red C.I. 16035 (erhältlich bei Firma Sigma-Aldrich, Art. Nr. 48,884-8) eingerührt werden und an Stelle von 1,51 g Glimmer die gleiche Menge Silicaflakes eingesetzt wird.

Die so erhaltenen getrockneten, beschichteten Silicaflakes weisen eine extrem hohe, metallisch wirkende Billanz, die das Produkt als attraktives Effektpigment qualifiziert, auf. Die verwendeten Ausgangsmaterialien sind kosmetisch unbedenklich und besitzen ein im Vergleich zu unbeschichteten Pigmenten verbessertes Hautgefühl.

### Beispiel 3

Zu 50 ml Wasser werden bei Raumtemperatur nacheinander 1,5 g Madurit® SMW 818 (Firma Solutia, Wiesbaden, Deutschland), 2 ml einer gesättigten Lösung von Zaponschwarz X50 (lipophiler Chromkomplex-Farbstoff, BASF AG Ludwigshafen, Deutschland), 2 ml 1-Methyl-2-pyrrolidon und 1,5 g Silica-Flakes zugegeben, gerührt und auf 70 °C erhitzt. Der Beschichtungsvorgang wird durch Zugabe von 3 ml einer 2 gew.-%igen Ameisensäurelösung ausgelöst. Es wird ein grau-schwarzes Pigment mit stark opaleszierenden Eigenschaften erhalten.

### Beispiel 4

Zu 50 ml Wasser werden bei Raumtemperatur nacheinander 1,5 g Madurit® SMW 818 (Firma Solutia, Wiesbaden, Deutschland), 0,03 g Viktoria Blau BO, 2 ml 1-Methyl-2-pyrrolidon und 1,5 g Silica-Flakes zugegeben, gerührt und auf 70 °C erhitzt. Der Beschichtungsvorgang wird durch Zugabe von 1 ml einer 2 gew.-%igen Ameisensäurelösung ausgelöst. Nach der Zugabe der Ameisensäure wird 30 min bei 70 °C nachgerührt. Es wird ein blaues Pigment mit hoher Brillanz und opaleszierenden Eigenschaften erhalten.

Zu 50 ml Wasser werden bei Raumtemperatur nacheinander 1,5 g Madurit® SMW 818 (Firma Solutia, Wiesbaden, Deutschland) und 0,75 g Glimmer zugegeben, gerührt und auf 70 °C erhitzt. Der Beschichtungsvorgang wird durch Zugabe von 1 ml einer 2 gew.-%igen Ameisensäurelösung ausgelöst. Nach der Zugabe der Ameisensäure wurde 30 min bei 70 °C nachgerührt. Das im Verhältnis zur Glimmermenge überschüssige Melamin-Formaldehyd-Harz wird in Form von im Wesentlichen runden monodispersen Kugeln auf der Oberfläche des Pigments abgeschieden (siehe Abbildung 2). Der Kugelanteil, zusammen mit der Kantenverrundung des beschichteten Substrats durch die Melamin-Formaldehyd-Harz-Belegung verleiht dem Produkt ein angenehmes Hautgefühl. Dies kann bereits beim Verreiben des Produkts zwischen den Fingern festgestellt werden.

### Beispiel 6

Zu 50 ml Wasser werden bei Raumtemperatur nacheinander 1,5 g Aluminium-Flakes (Firma Sigma-Aldrich, Art. Nr. 51,858-1), 1,5 g Madurit® SMW 818 (Firma Solutia, Wiesbaden, Deutschland) und 0,02 g Viktoria Blau BO, gerührt und auf 70 °C erhitzt. Der Beschichtungsvorgang wird durch Zugabe von 2,5 ml einer 2 gew.-%igen Ameisensäurelösung ausgelöst. Nach der Zugabe der Ameisensäure wird 30 min bei 70 °C nachgerührt. Es wird eine homogene blaue Beschichtung der Aluminium-Flakes erhalten.

### Beispiel 7

0,75 g Silica-Flakes werden in einer ammoniakalischen Silbernitrat-Lösung durch Zugabe von Glucose mit einer dünnen Silberschicht belegt. Die versilberten und gewaschenen Flakes werden in 25 ml Wasser suspendiert, mit 0,75 g Madurit® SMW 818 (Firma Solutia, Wiesbaden, Deutschland) und 0,03 g Methylorange versetzt, gerührt und auf 70 °C erhitzt. Der Beschichtungsvorgang wird durch Zugabe von 1,25 ml einer 2 gew.%igen Ameisensäurelösung ausgelöst. Es wird ein sehr attraktives gold-metallisches Pigment erhalten.

In einer ersten Stufe wird bei Raumtemperatur 1,5 g gemahlener Glimmer in 50 ml Wasser suspendiert, mit 1,5 g Madurit® SMW 818 (Firma Solutia, Wiesbaden, Deutschland) und 0,05 g Supracenviolett 3B versetzt; gerührt und auf 70 °C erhitzt. Der Beschichtungsvorgang wird durch Zugabe von 1 ml einer 2 gew.-%igen Ameisensäurelösung ausgelöst. Nach der Zugabe der Ameisensäure wird 30 min bei 70 °C nachgerührt. Es wird ein blau-violettes Pigment erhalten, welches gewaschen und in ein zweites Reaktionsgefäß überführt wird. Das in der ersten Stufe erhaltene Produkt wird in 50 ml Wasser suspendiert. 1,5 g Madurit® SMW 818 (Firma Solutia, Wiesbaden, Deutschland) und 0,1 g Blankophor PM (Fluoreszenzfarbstoff der Firma Bayer) werden zugeben. Anschließend wird unter Rühren auf 70 °C erhitzt. Der zweite Beschichtungsvorgang wird durch Zugabe von 1,5 ml einer 2 gew.-%igen Ameisensäurelösung ausgelöst. Nach der Zugabe der Ameisensäure wird 30 min bei 70 °C nachgerührt. Das erhaltene Produkt zeigt gegenüber dem Produkt der ersten Stufe ein helleres Blau-violett als Grundfarbe, verbunden mit einer durch die Zweitbeschichtung erhaltenen Fluoreszenz im UV-Licht bei 365 nm.

### Beispiel 9

1 g eines mit Allura Rot (C.I: 16035) und Melamin-Formaldehyd-Harz beschichteten Glimmers (Pigment aus Beispiel 2) wird bei 105 °C und 200 mbar im Vakuumtrockenschrank 24 h getrocknet. Danach wird das Pigment in 50 ml trockenem Toluol suspendiert. Zu dieser Suspension werden unter Rühren mit einem Magnetrührer 0,5 ml getrocknetes Pyridin und 0,5 ml Stearinsäurechlorid gegeben. Die Mischung wird 3 Stunden bei 60 °C gerührt. Danach wird der Ansatz auf 200 ml Wasser gegossen und die organische Phase abgetrennt. Die wässrige Phase wird zweimal mit 50 ml Toluol gewaschen, um nicht umgesetztes Säurechlorid bzw. hydrolysiertes Säurechlorid zu entfernen. Der Bodensatz der wässrigen Phase wird abgetrennt, dann mit einer Mischung aus Wasser und Ethanol gewaschen und bei 105 °C im Vakuumtrockenschrank getrocknet.

Mit dem getrockneten, hydrophob funktionalisierten Farbpigment werden Benetzungsversuche in Wasser und Toluol durchgeführt. Im Vergleich zu dem nicht funktionalisierten Farbpigment mit Wasser ein deutlich hydrophobes Verhalten. Bei der Benetzung mit Toluol besitzt das mit Stearinsäure funktionalisierte Farbpigment hingegen eine deutlich bessere Benetzbarkeit.

### Beispiel 10

Zu einem Ansatz bestehend aus 1,5 g F-Glimmer (Glimmer einer Dicke von ca. 1 µm und einem Durchmesser von etwa 10-30 µm; Merck KGaA), 1,5 g Madurit SMW 818 (Solutia AG), 50 ml Wasser, 0,1 ml Blankophor P flüssig (optischer Aufheller; Bayer AG) und 0,5 mg Rhodamin B (Fluoreszenzfarbstoff; Merck KGaA) wird unter kräftigem Rühren bei 70 °C 1 ml einer 2 %igen Ameisensäure zugegeben. Der Beschichtungsvorgang des Glimmers mit den beiden Farbstoffen ist nach ca. 15 min beendet. Der Feststoff wird abgesaugt, gewaschen und getrocknet. Das erhaltene Produkt zeigt eine kräftig rote. Fluoreszenz bei Anregung mit Licht einer Wellenlänge von 366 nm.

Die nachfolgenden Vergleichsbeispiele 1 und 2 zeigen, dass die Einzelfarbstoffe diese Eigenschaft nicht besitzen.

### Vergleichsbeispiel 1

Zu einem Ansatz bestehend aus 1,5 g F-Glimmer (Glimmer einer Dicke von ca. 1 µm und einem Durchmesser von etwa 10-30 µm; Merck KGaA), 1,5 g Madurit SMW 818 (Solutia AG), 50 ml Wasser und 0,1 ml Blankophor P flüssig (optischer Aufheller; Bayer AG) wird unter kräftigem Rühren bei 70 °C 1 ml einer 2 %igen Ameisensäure zugegeben. Der Beschichtungsvorgang des Glimmers mit Blankophor P ist nach ca. 15 min beendet. Der Feststoff wird abgesaugt, mit de-ionisiertem Wasser gewaschen und getrocknet. Das erhaltene Produkt zeigt eine kräftige, blau-weiße Fluoreszenz bei Anregung mit Licht einer Wellenlänge von 366 nm.

Zu einem Ansatz bestehend aus 1,5 g F-Glimmer, 1,5 g Madurit SMW 818 (Solutia AG), 50 ml Wasser und 0,5 mg Rhodamin B (Fluoreszenzfarbstoff; Merck KGaA) wird unter kräftigem Rühren bei 70 °C 1 ml einer 2 %igen Ameisensäure zugegeben. Der Beschichtungsvorgang des Glimmers mit Rhodamin B ist nach ca. 15 min beendet. Der Feststoff wird abgesaugt, gewaschen und getrocknet. Das erhaltene Produkt zeigt eine sehr schwache, rote Fluoreszenz bei Anregung mit Licht einer Wellenlänge von 366 nm.

### Beispiel 11

Zu einem Ansatz bestehend aus 1,5 g F-Glimmer (Merck), 1,5 g Madurit SMW 818 (Solutia AG), 50 ml Wasser, 0,1 ml Blankophor P flüssig (optischer Aufheller, Bayer AG) und 0,5 mg Acridine (Fluoreszenzfarbstoff; Aldrich) wird unter kräftigem Rühren bei 70 °C 1 ml einer 2 %igen Ameisensäure zugegeben. Der. Beschichtungsvorgang des Glimmers mit den beiden Farbstoffen ist nach ca. 15 min. beendet. Der Feststoff wird abgesaugt, mit de-ionisiertem Wasser gewaschen und getrocknet. Das erhaltene Produkt zeigt eine neutral-weiße Fluoreszenz bei Anregung mit Licht einer Wellenlänge von 366 nm.

Das Ergebnis ohne Acridine ist in Vergleichsbeispiel 1 bereits beschrieben (blau-weiße Fluoreszenz).

Der gleiche Ansatz wie in Beispiel 11 ohne Blankophor P flüssig führt zu einem Produkt ohne Fluoreszenzeigenschaften bei Anregung mit Licht einer Wellenlänge von 366 nm.

## Patentansprüche

1. Beschichtetes plättchenförmiges Trägermaterial, **dadurch gekennzeichnet, dass** das Trägermaterial aus einem anorganischen Substrat besteht und das Trägermaterial mit mindestens einer Beschichtung versehen ist, wobei Jede Schicht mindestens ein gehärtetes Melamin-Formaldehyd-Harz enthält oder aus einem solchen besteht, und wobei das gehärtete Melamin-Formaldehyd-Harz einen oder mehrere anorganische oder organische Farbstoffe und/oder einen oder mehrere anorganische oder organische UV-Absorber enthält, wobei die Farbstoffe im Medium, In welchem das Pigment beschichtet wird löslich sind.

2. Beschichtetes plättchenförmiges Trägermaterial gemäß Anspruch 1, **dadurch gekennzeichnet, dass** das anorganische Trägermaterial aus der Gruppe bestehend aus "Glimmer, Silikaflakes, Glasflakes, Periglanzpigmenten und Metallflakes oder -follen" gewählt ist.

3. Beschichtetes plättchenförmiges Trägermaterial gemäß Anspruch 2, **dadurch gekennzeichnet, dass** die Metallflakes oder -folien aus Silber, Kupfer, Nickel, Gold, Aluminium oder Legierungen dieser Metalle bestehen.

4. Beschichtetes plättchenförmiges Trägermaterial gemäß Anspruch 1,2 oder 3, **dadurch gekennzeichnet, dass** das anorganische Substrat eine metallische Beschichtung aufweist.

5. Beschichtetes plättchenförmlges Trägermaterial gemäß Anspruch 4, **dadurch gekennzeichnet, dass** die metallische Beschichtung aus Silber, Kupfer, Nickel, Gold, Aluminium oder Legierungen dieser Metalle besteht.

6. Beschichtetes plättchenförmiges Trägermaterial gemäß einem oder mehreren der Ansprüche 1 bis 5, **dadurch gekennzeichnet, dass** der oder die Farbstoffe In einer oder mehreren, Inneren, Melamin-Formaidehyd-Harz enthaltenden Schichten, enthalten sind und der oder die UV-Absorber In einer oder mehreren äußeren, Melamln-Formaldehyd-Harz enthaltenden Schichten, enthalten sind.

7. Beschichtetes plättchenförmiges Trigermaterial gemäß einem oder mehreren der Ansprüche 1 bis 6, **dadurch gekennzeichnet, dass** auf der äußersten Beschichtung zusätzlich, Im Wesentlichen runde gehärtete Melamin-Formaldehydharzpartlkel aufgebracht sind, die einen oder mehrere Farbstoffe und/oder einen oder mehrere UV-Absorber enthalten oder aber frei von Farbstoffen und/oder UV-Absorbern sind.

8. Beschichtetes plättchenförmiges Trägermatedal gemäß einem oder mehreren der Ansprüche 1 bis 7, **dadurch gekennzeichnet, dass** das gehärtete Melamin-Formaldehyd-Harz der äußersten Schicht mit funktionellen Gruppen modifiziert ist.

9. Beschichtetes plättchenförmiges Trägermaterial gemäß Anspruch 8, **dadurch gekennzeichnet, dass** die funktionellen Gruppen, welche die äußerste Schicht modifizieren über eine aminofunktionelle Verbindung, die neben der Aminogruppe eine oder mehrere weitere funktionelle Gruppen aufweist, eingeführt wird, In dem diese aminofunktionelle Verbindung an der Polykondensationsreaktion zwischen Melamin und Formaldehyd teilnimmt und über die Aminofunktion In das Melamin-Formaldehyd-Netzwerk eingebaut wird und wobei die somit an die Oberfläche gebrachten funktionellen Gruppen gegebenenfalls weiter modifiziert werden.

10. Beschichtetes plättchenförmiges Trägermaterial gemäß Anspruch 8, **dadurch gekennzeichnet, dass** das gehärtete Melamin-Formaldehyd-Harz der äußersten Schicht über die in diesem vorhandenen Methylolamin- oder Aminogruppen mit gegenüber Hydroxy- und/oder Aminogruppen reaktiven Verbindungen unter Funktionallsierung der Oberfläche modifiziert ist.

11. Beschichtetes plättchenförmiges Trägermaterial gemäß einem oder mehreren der Ansprüche 1 bis 11, wobei als Farbstoffe mindestens ein Fluoreszenzfarbstoff und ein weiterer gegebenenfalls fluoreszierender Farbstoff im Melamin-Formaldehyd-Harz enthalten Ist, wobei der weitere Farbstoff in einer Menge enthalten Ist, die dem Pigment bei alleinigem Einsatz dieses Farbstoffs im wesentlichen keine Farbe oder Fluoreszenz verleiht.

12. Verfahren zur Herstellung eines einfach oder mehrfach beschichteten plättchenförmigen Trägermaterials, gemäß einem oder mehreren der Ansprüche 1 bis 11, **dadurch gekennzeichnet, dass**
bei einer Einfachbaschichtung
In einem ersten Schritt ein anorganisches plättchenförmiges Substrat in basischem wässrigem Milieu enthaltend Melamin und Formaldehyd und/oder Methylolmelamin, welches gegebenenfalls alkoxyliert sein kann, suspendiert wird und
in einem zweiten Schritt durch Senkung des pH-Werts In den sauren Bereich eine Vernetzung der organischen Bestandteile herbeigeführt wird
und bei einer Mehrfachbeschichtung
die Schritte eins und zwei mit dem Produkt der vorangegangenen Beschichtungsreaktion wiederholt werden.

13. Verfahren gemäß Anspruch 12, **dadurch gekennzeichnet, dass** ein Teil des Melamins durch andere vernetzende Moleküle aus der Gruppe bestehend aus "Guanaminen, Phenolen und Harnstoffen" ersetzt Ist und/oder ein Teil des Methylolmelamins durch entsprechende Guanamin-, Phenol- oder Harnstoffanaloga ersetzt ist.

14. Verfahren gemäß Anspruch 12 oder 13, **dadurch gekennzeichnet, dass** vor Einsetzen oder während der Vernetzung anorganische oder organische Farbstoffe und/oder anorganische oder organische UV-Absorber zugesetzt werden.

15. Verfahren gemäß Anspruch 14, **dadurch gekennzeichnet, dass** als Farbstoffe mindestens ein Fluoreszenzfarbstoff und ein weiterer gegebenenfalls fluoreszierender Farbstoff zugesetzt wird, wobei der weitere Farbstoff in einer Menge zugesetzt wird, die dem Pigment bei alleinigem Zusatz dieses Farbstoffs Im wesentlichen keine Farbe oder Fluoreszenz verleiht.

16. Verfahren gemäß einem oder mehreren der Ansprüche 12 bis 14, **dadurch gekennzeichnet, dass** die Senkung des pH-Werts In den sauren Bereich durch Oxidation von überschüssigem und/oder nicht umgesetztem und/oder In den Methylolmelaminen enthaltenem Formaldehyd mittels Wasserstoffperoxid bewirkt wird.

17. Verfahren gemäß einem oder mehreren der Ansprüche 12 bis 16, **dadurch gekennzeichnet, dass** beim letzten Beschichtungsschritt neben Melamin und Formaldehyd und/oder Methylolmelamin eine aminofunktionelle Verbindung, die neben der Aminogruppe eine oder mehrere funktionelle Gruppen aufweist, an der Polykondensationsreaktion tellnimmt, wobei die aminofunktionelle Verbindung über die Aminofunktion In das Melamin-Formaldehyd-Netzwerk eingebaut wird und wobei die somit an die Oberfläche gebrachten funktionellen Gruppen gegebenenfalls weiter modifiziert werden.

18. Verfahren nach einem oder mehreren der Ansprüche 12 bis 16, **dadurch gekennzeichnet, dass** das gehärtete Melamin-Formaldehyd-Harz der äußersten Schicht über die an deren Oberfläche vorhandenen Methylolamin- oder Aminogruppen mit Verbindungen umgesetzt wird, die eine gegenüber Hydroxy- und/oder Aminogruppen reaktive Gruppe, neben einer oder mehreren weiteren funktionellen Gruppen, aufweisen.

19. Verwendung eines oder mehrerer der beschichteten plättchenförmigen Trägermaterialien der Ansprüche 1 bis 11 als Effektpigmente in Farben, Lacken, Druckfarben, Kunststoffen, Pulverlacken, zur Saatguteinfärbung, in kosmetischen Formulierungen und/oder zur Pigmentierung von Lebensmitteln.

20. Verwendung gemäß Anspruch 19 zum Zweck der Markierung und/oder Kodierung von Produkten.

21. Zusammensetzungen enthaltend eines oder mehrere der beschichteten plättchenförmigen Trägermaterialien der Ansprüche 1 bis 11 als Effektpigment.

## Claims

1. Coated platelet-shaped carrier material, **characterized in that** the carrier material is composed of an inorganic substrate and is provided with at least one coating, each layer comprising at least one cured melamine-formaldehyde resin or being composed of such a resin, and the cured melamine-formaldehyde resin comprising one or more organic or inorganic dyes and/or one or more organic or inorganic UV absorbers, the dyes being soluble in the medium in which the pigment is coated.

2. Coated platelet-shaped carrier material according to Claim 1, **characterized in that** the inorganic carrier material is selected from the group consisting of mica, silica flakes, glass flakes, pearlescent pigments, metal flakes and metal foils.

3. Coated platelet-shaped carrier material according to Claim 2, **characterized in that** the metal flakes or metal foils are composed of silver, copper, nickel, gold, aluminium or alloys of these metals.

4. Coated platelet-shaped carrier material according to Claim 1, 2 or 3, **characterized in that** the inorganic substrate has a metallic coating.

5. Coated platelet-shaped carrier material according to Claim 4, **characterized in that** the metallic coating is composed of silver, copper, nickel, gold, aluminium or alloys of these metals.

6. Coated platelet-shaped carrier material according to one or more of Claims 1 to 5, **characterized in that** the dye or dyes is or are present in one or more inner layers comprising melamine-formaldehyde resin and the UV absorber or absorbers is or are present in one or more outer layers comprising melamine-formaldehyde resin.

7. Coated platelet-shaped carrier material according to one or more of Claims 1 to 6, **characterized in that** substantially spherical cured melamine-formaldehyde resin particles which comprise one or more dyes and/or one or more UV absorbers or else are free from dyes and/or UV absorbers are additionally applied to the outermost coating.

8. Coated platelet-shaped carrier material according to one or more of Claims 1 to 7, **characterized in that** the cured melamine-formaldehyde resin of the outermost layer is modified with functional groups.

9. Coated platelet-shaped carrier material according to Claim 8, **characterized in that** the functional groups which modify the outermost layer are introduced by way of an amino-functional compound which in addition to the amino group contains one or more further functional groups, this amino-functional compound participating in the polycondensation reaction between melamine and formaldehyde and being incorporated into the melamine-formaldehyde network by way of the amino function, with the functional groups brought to the surface in this way being modified further where appropriate.

10. Coated platelet-shaped carrier material according to Claim 8, **characterized in that** the cured melamine-formaldehyde resin of the outermost layer is surface-functionalizing modified with compounds reactive towards hydroxyl and/or amino groups by way of the methylolamine or amino groups present in the said resin.

11. Coated platelet-shaped carrier material according to one or more of Claims 1 to 10, the melamine-formaldehyde resin comprising as dyes at least one fluorescent dye and one further, optionally fluorescent dye, the further dye being present in an amount which gives the pigment essentially no colour or fluorescence when this dye is used alone.

12. Process for producing a singly or multiply coated platelet-shaped carrier material, according to one or more of Claims 1 to 11, **characterized in that** it comprises, in the case of a single coating,
a first step in which an inorganic platelet-shaped substrate is suspended in a basic aqueous medium, comprising melamine and formaldehyde and/or methylolmelamine, which may optionally have been alkoxylated, and
a second step in which crosslinking of the organic constituents is brought about by lowering the pH into the acidic range,
and, in the case of a multiple coating,
repeating the first and second steps with the product of the preceding coating reaction.

13. Process according to Claim 12, **characterized in that** some of the melamine is replaced by other crosslinking molecules from the group consisting of guanamines, phenols and ureas and/or some of the methylolmelamine is replaced by corresponding guanamine, phenol or urea analogues.

14. Process according to Claim 12 or 13, **characterized in that**, before the onset of or during crosslinking, organic or inorganic dyes and/or organic or inorganic UV absorbers are added.

15. Process according to Claim 14, **characterized in that** dyes added comprise at least one fluorescent dye and one further, optionally fluorescent dye, the further dye being added in an amount which gives the pigment essentially no colour or fluorescence when this dye is used alone.

16. Process according to one or more of Claims 12 to 14, **characterized in that** the lowering of the pH into the acidic range is brought about by oxidation of excess and/or unreacted formaldehyde and/or formaldehyde present in the methylolmelamines, by means of hydrogen peroxide.

17. Process according to one or more of Claims 12 to 16, **characterized in that** in the final coating step, in addition to melamine and formaldehyde and/or methylolmelamine, an amino-functional compound which in addition to the amino group contains one or more functional groups participates in the polycondensation reaction, the amino-functional compound being incorporated into the melamine-formaldehyde network by way of the amino function, and with the functional groups brought to the surface in this way being modified further where appropriate.

18. Process according to one of or more of Claims 12 to 16, **characterized in that** the cured melamine-formaldehyde resin of the outermost layer is reacted by way of the methylolamine or amino groups present on its surface with compounds which contain a group which is reactive towards hydroxyl and/or amino groups, in addition to one or more further functional groups.

19. Use of one or more of the coated platelet-shaped carrier materials of Claims 1 to 11 as effect pigments in paints, varnishes, printing inks, plastics, powder coating materials, for colouring seed, in cosmetic formulations and/or for pigmenting foods.

20. Use according to Claim 19 for the purpose of marking and/or coding products.

21. Compositions comprising one or more of the coated platelet-shaped carrier materials of Claims 1 to 11 as effect pigment.

## Revendications

1. Matériau de support en forme de plaquettes, enrobé, **caractérisé en ce que** le matériau de support consiste en un support inorganique et le matériau de support est muni d'au moins un enrobage, chaque couche contenant au moins une résine mélamine-formaldéhyde durcie ou consistant en une telle résine, et la résine mélamine-formaldéhyde durcie contenant un ou plusieurs colorants et/ou un ou plusieurs absorbeurs UV organiques ou inorganiques, les colorants étant solubles dans le milieu dans lequel le pigment est enrobé.

2. Matériau de support en forme de plaquettes, enrobé, selon la revendication 1, **caractérisé en ce que** le matériau de support inorganique est choisi dans le groupe constitué par "le mica, les paillettes de mica, les paillettes de verre, les pigments nacrés et les paillettes ou pellicules métalliques".

3. Matériau de support en forme de plaquettes, enrobé, selon la revendication 2, **caractérisé en ce que** les paillettes ou pellicules métalliques sont constituées d'argent, de cuivre, de nickel, d'or, d'aluminium ou d'alliages de ces métaux.

4. Matériau de support en forme de plaquettes, enrobé, selon la revendication 1, 2 ou 3, **caractérisé en ce que** le support inorganique comporte un enrobage métallique.

5. Matériau de support en forme de plaquettes, enrobé, selon la revendication 4, **caractérisé en ce que** l'enrobage métallique est constitué d'argent, de cuivre, de nickel, d'or, d'aluminium ou d'alliages de ces métaux.

6. Matériau de support en forme de plaquettes, enrobé, selon une ou plusieurs des revendications 1 à 5, **caractérisé en ce que** le ou les colorants sont contenus dans une ou plusieurs couches internes contenant de la résine mélamine-formaldéhyde et l'absorbeur UV ou les absorbeurs UV est/sont contenu(s) dans une ou plusieurs couches externes contenant de la résine mélamine-formaldéhyde.

7. Matériau de support en forme de plaquettes, enrobé, selon une ou plusieurs des revendications 1 à 6, **caractérisé en ce que** sur l'enrobage le plus externe sont en outre appliquées des particules de résine mélamine-formaldéhyde durcie, pratiquement rondes, qui contiennent un ou plusieurs colorants et/ou un ou plusieurs absorbeurs UV ou bien sont exemptes de colorants et/ou d'absorbeurs UV.

8. Matériau de support en forme de plaquettes, enrobé, selon une ou plusieurs des revendications 1 à 7, **caractérisé en ce que** la résine mélamine-formaldéhyde durcie de la couche la plus externe est modifiée par des groupes fonctionnels.

9. Matériau de support en forme de plaquettes, enrobé, selon la revendication 8, **caractérisé en ce que** les groupes fonctionnels qui modifient la couche la plus externe sont introduits au moyen d'un composé à fonction amino qui, outre le groupe amino, comporte un ou plusieurs autres groupes fonctionnels, ce composé à fonction amino participant à la réaction de polycondensation entre mélamine et formaldéhyde et étant incorporé par l'intermédiaire de la fonction amino dans le réseau mélamine-formaldéhyde, et les groupes fonctionnels ainsi amenés à la surface étant éventuellement modifiés davantage.

10. Matériau de support en forme de plaquettes, enrobé, selon la revendication 8, **caractérisé en ce que** la résine mélamine-formaldéhyde durcie de la couche la plus externe est modifiée, par l'intermédiaire des groupes amino ou méthylolamine présents dans celle-ci, par des composés réactifs vis-à-vis de groupes amino et/ou hydroxy, avec fonctionnalisation de la surface.

11. Matériau de support en forme de plaquettes, enrobé, selon une ou plusieurs des revendications 1 à 10, dans lequel, en tant que colorants, au moins un colorant fluorescent et un autre colorant éventuellement fluorescent sont contenus dans la résine mélamine-formaldéhyde, l'autre colorant étant contenu en une quantité qui ne confère au pigment pratiquement aucune couleur ni fluorescence lorsque ce colorant est utilisé seul.

12. Procédé pour la préparation d'un matériau de support en forme de plaquettes, une ou plusieurs fois enrobé, selon une ou plusieurs des revendications 1 à 11, **caractérisé en ce que**
dans le cas d'un enrobage en une fois,
dans une première étape on met en suspension un support inorganique, sous forme de plaquettes, dans un milieu aqueux basique contenant de la mélamine et du formaldéhyde et/ou de la méthylolmélamine qui peut éventuellement être alcoxylée, et
dans une deuxième étape on provoque une réticulation des composants organiques par abaissement du pH dans le domaine acide
et dans le cas d'un enrobage en plusieurs fois
on répète les étapes un et deux avec le produit de la précédente réaction d'enrobage.

13. Procédé selon la revendication 12, **caractérisé en ce qu'**on remplace une partie de la mélamine par d'autres molécules réticulables choisies dans le groupe constitué par "les guanamines, phénols et urées" et/ou une partie de la méthylolmélamine par des analogues correspondants de guanamine, phénol et urée.

14. Procédé selon la revendication 12 ou 13, **caractérisé en ce qu'**avant la mise en réaction ou pendant la réticulation on ajoute des colorants organiques ou inorganiques et/ou des absorbeurs UV organiques ou inorganiques.

15. Procédé selon la revendication 14, **caractérisé en ce qu'**on ajoute en tant que colorants au moins un colorant fluorescent et un autre colorant éventuellement fluorescent, l'autre colorant étant ajouté en une quantité qui ne confère au pigment pratiquement aucune couleur ni fluorescence lorsque ce colorant est ajouté seul.

16. Procédé selon une ou plusieurs des revendications 12 à 14, **caractérisé en ce que** l'abaissement du pH dans le domaine acide est provoqué par oxydation, au moyen de peroxyde d'hydrogène, du formaldéhyde en excès et/ou n'ayant pas réagi et/ou contenu dans les méthylolmélamines.

17. Procédé selon une ou plusieurs des revendications 12 à 16, **caractérisé en ce que** dans la dernière étape d'enrobage, outre la mélamine et le formaldéhyde et/ou la méthylolmélamine, un composé à fonction amino, qui, outre le groupe amino, comporte un ou plusieurs groupes fonctionnels, participe à la réaction de polycondensation, le composé à fonction amino étant incorporé par l'intermédiaire de la fonction amino dans le réseau mélamine-formaldéhyde, et les groupes fonctionnels ainsi amenés à la surface étant éventuellement modifiés davantage.

18. Procédé selon une ou plusieurs des revendications 12 à 16, **caractérisé en ce que** la résine mélamine-formaldéhyde durcie de la couche la plus externe est mise en réaction, par l'intermédiaire des groupes amino ou méthylolamine présents à sa surface, avec des composés qui comportent, en plus d'un ou plusieurs autres groupes fonctionnels, un groupe réactif vis-à-vis de groupes hydroxy et/ou amino.

19. Utilisation d'un ou plusieurs des matériaux de support en forme de plaquettes, enrobés, des revendications 1 à 11, en tant que pigments à effet dans des peintures, des vernis, des encres d'imprimerie, des matières plastiques, des peintures en poudre, pour la coloration de semences, dans des compositions cosmétiques et/ou pour la pigmentation de produits alimentaires.

20. Utilisation selon la revendication 19, dans le but du marquage et/ou du codage de produits.

21. Compositions contenant un ou plusieurs des matériaux de support en forme de plaquettes, enrobés, des revendications 1 à 11, en tant que pigment à effet.
